Europäisches Patentamt

European Patent Office

Office européen des brevets

(19)

(11) **EP 0 830 601 B1**

(12) **FASCICULE DE BREVET EUROPEEN**

(45) Date de publication et mention
de la délivrance du brevet:
**27.10.1999 Bulletin 1999/43**

(51) Int Cl.$^6$: **G01N 33/533**, G01N 33/542,
G01N 33/58, C07K 19/00,
C07K 14/195

(21) Numéro de dépôt: **96922078.9**

(22) Date de dépôt: **07.06.1996**

(86) Numéro de dépôt international:
**PCT/FR96/00865**

(87) Numéro de publication internationale:
**WO 96/42016 (27.12.1996 Gazette 1996/56)**

(54) **UTILISATION D'UN COMPLEXE PHYCOBILIPROTEINE-PEPTIDE DE LIAISON EN TANT QUE TRACEUR FLUORESCENT**

VERWENDUNG EINER PHYCOBILIPROTEIN-PEPTIDKOMPLEXVERBINDUNG ALS FLUORESZENZLABEL

USE OF A PHYCOBILIPROTEIN-LINKER PEPTIDE COMPLEX AS A FLUORESCENT TRACER

(84) Etats contractants désignés:
**AT BE CH DE DK ES FI FR GB GR IE IT LI LU MC
NL PT SE**

(30) Priorité: **09.06.1995 FR 9506821**

(43) Date de publication de la demande:
**25.03.1998 Bulletin 1998/13**

(73) Titulaire: **CIS BIO INTERNATIONAL
91400 Saclay (FR)**

(72) Inventeur: **MATHIS, Gérard
F-30200 Bagnols-sur-Cèze (FR)**

(74) Mandataire: **Gillard, Marie-Louise et al
Cabinet Beau de Loménie,
158, rue de l'Université
75007 Paris (FR)**

(56) Documents cités:
**US-A- 4 857 474**

**Description**

L'invention concerne l'utilisation d'un complexe phycobiliprotéine-peptide de liaison en tant que traceur fluorescent, notamment dans un procédé de détection et/ou de détermination par fluorescence d'un analyte dans un milieu susceptible de le contenir, ainsi que les conjugués fluorescents constitués dudit complexe lié de manière covalente à l'un des éléments d'un couple de liaison spécifique ligand/récepteur.

L'utilisation de dosages immunologiques pour l'analyse qualitative et quantitative de composés dans des fluides biologiques est à l'heure actuelle largement répandue.

Parmi les techniques existantes, les dosages par fluorimétrie ont pris une importance croissante.

En effet, ils présentent un certain nombre d'avantages parmi lesquels la sensibilité, la rapidité de la mesure, la stabilité et l'inocuité des réactifs marqués par des composés fluorescents et le coût relativement réduit.

Les phycobiliprotéines sont des constituants du phycobilisome de différentes bactéries, algues ou cryptomonades et sont en général de quatre types : les phycocyanines, les phycoerythrines, les phycoerythrocyanines et les allophycocyanines.

Elles sont formées de sous-unités $\alpha$ et $\beta$, et parfois $\gamma$ portant chacune un ou plusieurs fluorophores, et sont isolées principalement sous forme de trimères ou d'hexamères.

Certaines d'entre elles sont utilisées comme marqueurs fluorescents en raison de nombreux avantages qu'elles présentent en terme de rendement quantique, bandes d'absorption, stabilité et solubilité (V.OI et al, J. Cell Biology 1982-93-981).

Schématiquement, les phycobilisomes sont formés de deux parties :

- le coeur formé d'éléments cylindriques constitués par des allophycocyanines ;
- des bâtonnets, formés d'éléments cylindriques, fixés au coeur, lesdits éléments étant constitués par des phycoerythrines et des phycocyanines.

Les bâtonnets et les cylindres sont formés par un assemblage de disques de phycobiliprotéines. Ces disques sont assemblés entre eux ainsi que les bâtonnets au coeur et le coeur à la membrane thylakoïde par des peptides de liaison.

Ces peptides sont nommés en fonction de leur localisation selon la publication A. Glazer, J. Biol. Chem., 1989, 264, 1-4:

$L_R$    pour peptide de liaison dans le bâtonnet
$L_C$    pour peptide de liaison dans le coeur
$L_{RC}$    pour peptide de liaison dans le bâtonnet-coeur
$L_{CM}$    pour peptide de liaison dans le coeur-membrane.

La purification des phycobiliprotéines par des méthodes conventionnelles permet l'obtention de complexes trimériques ou hexamériques $(\alpha\beta)_3$ ou $(\alpha\beta)_6$ dépourvus de peptides de liaison. Les trimères ont une forme discoïdale de $\approx 30$ Å d'épaisseur et de $\approx 120$ Å de diamètre.

L'utilisation de phycobiliprotéines, notamment l'allophycocyanine et la phycoerythrine, dans des dosages immunologiques par fluorimétrie est décrite notamment dans EP 0 174 744 et 0 076 695 ainsi que dans les brevets US 4 520 110 et 4 542 104.

Il a été trouvé que dans certaines conditions de purification des phycobilisomes, il était possible d'obtenir des complexes phycobiliprotéine-peptide de liaison (P. Fuglistaller et al., Biol. Chem. Hoppe-Seyler, 1987, 368, 353-367). Néanmoins, ces complexes étaient jusqu'à maintenant décrits dans la littérature comme étant relativement peu stables et sensibles aux protéases (W. Reuter et C. Nickel-Reuter, J. Photochem. Photobiol., B : Biol., 1993, 18, 51-66).

De manière avantageuse, on a maintenant trouvé que ces complexes présentaient des particularités spectroscopiques intéressantes par rapport aux phycobiliprotéines pour une utilisation en tant que traceur fluorescent.

En effet, un tel complexe phycobiliprotéine-peptide de liaison possède toujours des propriétés spectroscopiques différentes de celles de la phycobiliprotéine seule, avec en général :

- augmentation du rendement quantique
et/ou - déplacement de la longueur d'onde d'émission
et/ou - modification de la longueur d'onde d'absorption
et/ou - modification du coefficient d'absorption molaire

par rapport à la phycobiliprotéine seule.

Ces propriétés peuvent se révéler particulièrement intéressantes lors de la mise en oeuvre d'un système de détection utilisant un ou plusieurs traceurs fluorescents, dans lequel, outre la stabilité des traceurs dans le milieu, deux paramètres sont d'importance capitale :

- le rendement quantique qui influe directement sur la limite de détection du système,
- la longueur d'onde d'émission qui lors de l'utilisation de plusieurs traceurs est un élément déterminant de leur choix.

De façon inattendue, on a maintenant trouvé que :

- ces complexes phycobiliprotéine-peptide de liaison étaient stables en solution et en présence de sérums de différentes origines contenant naturellement différentes protéases,
- il était possible de fixer de manière covalente ces complexes sur différentes protéines et anticorps tout en conservant les propriétés de fluorescence des complexes.

Dans un aspect avantageux, la phycobiliprotéine utilisée selon l'invention est choisie parmi la phycoerythrine, la phycoerythrocyanine, la phycocyanine, l'allophycocyanine et l'allophycocyanine B.

Dans la suite de la description, on utilisera les abréviations suivantes pour désigner les phycobiliprotéines préférées : allophycocyanine = AP, phycoerythrine = PE, phycoerythrocyanine = PEC, phycocyanine = PC, allophycocyanine B = APB

De préférence, le complexe phycobiliprotéine-peptide de liaison est extrait d'une cyanobactérie choisie parmi *Mastigoclodus Laminosus, Synechocystis 6701, Synechococcus 6301, Anabaena variabilis* et *Nostoc spec..*

Le peptide de liaison du complexe utilisé aux fins de l'invention est de préférence choisi parmi les peptides $L_R$, $L_C$, $L_{RC}$ et $L_{CM}$, tels que définis plus haut.

De manière avantageuse, les complexes utilisables selon l'invention sont définis de la manière suivante, à partir des phycobiliprotéines telles que désignées par les abréviations mentionnées ci-dessus, des sous-unités $\alpha$ et $\beta$ et des peptides de liaison mentionnés plus haut :

$$(\alpha^{PEC}, \beta^{PEC})_6 \, L_R, \, (\alpha^{PEC}, \beta^{PEC})_3 \, L_R,$$

$$(\alpha^{PC}, \beta^{PC})_6 \, L_R, \, (\alpha^{PC}, \beta^{PC})_6 \, L_{RC}, \, (\alpha^{PC}, \beta^{PC})_3 \, L_R,$$

$$(\alpha^{AP}, \beta^{AP})_3 \, L_C, \, (\alpha^{APB}, \alpha_2^{AP}, \beta_3^{AP}) \, L_C \text{ et } (\alpha^{AP}, \beta^{AP})_2 \, L_{CM}.$$

Avantageusement, on utilisera selon l'invention les complexes phycobiliprotéine-peptides de liaison en combinaison avec un ou plusieurs autres traceurs fluorescents.

Le complexe préféré aux fins de l'invention est le complexe $(\alpha^{AP}, \beta^{AP})_3 \, L_C$, c'est-à-dire un complexe phycobiliprotéine-peptide de liaison constitué d'un trimère de sous-unités $\alpha$ et $\beta$ d'allophycocyanine et du peptide de liaison dans le coeur.

La longueur des peptides varie suivant l'espèce et les processus de dégradation durant la purification. Elle est de préférence comprise entre 5000 et 30000.

Selon un aspect ultérieur, l'invention concerne également un procédé homogène de détection et/ou de détermination par fluorescence d'un analyte dans un milieu susceptible de le contenir, par mise en évidence du produit de la réaction entre l'analyte et au moins un récepteur correspondant, consistant :

1) à ajouter audit milieu un premier réactif constitué d'au moins un récepteur dudit analyte,

2) à ajouter un second réactif choisi parmi l'analyte ou au moins l'un de ses récepteurs, l'un des deux réactifs étant couplé avec un composé fluorescent donneur constitué par un cryptate, un chélate ou un complexe macrocyclique de terre rare et l'autre réactif étant couplé avec un composé fluorescent accepteur, l'ordre d'ajout des réactifs pouvant être inversé et, après excitation du mélange par une source de lumière à la longueur d'onde d'excitation du composé fluorescent donneur,

3) à mesurer le signal d'émission du composé fluorescent accepteur,

caractérisé en ce qu'on utilise comme composé fluorescent accepteur un complexe phycobiliprotéine-peptide de liaison tel que défini ci-dessus.

Dans la présente description, on définit par :

- "analyte" : toute substance ou groupe de substances, ainsi que ses ou leurs analogues, que l'on souhaite détecter et/ou déterminer;
- "récepteur" : toute substance capable de se fixer spécifiquement à un site dudit analyte;

- "ligand" : toute substance capable de se lier spécifiquement à un récepteur.

Des cryptates de terre rare utilisables dans un tel procédé de dosage ainsi que dans les méthodes par excès ou par compétition décrites ci-après sont notamment décrits dans les demandes EP 0 180 492, EP 0 232 348, EP 0 321 353 ou WO90/04791.

Des complexes macrocycliques de terre rare portant des groupes N-oxy sont également décrits dans la demande WO93/05049.

Ces cryptates et complexes macrocycliques de terre rare présentent l'avantage d'être très stables en milieu protéique et salin, cette propriété étant particulièrement importante dans le cas des immunoessais homogènes.

On utilisera de préférence en tant que composé fluorescent donneur dans les procédés et méthodes mentionnées dans la présente description un chélate , un cryptate ou un complexe macrocyclique portant des groupes N-oxy de terbium ou d'europium.

Selon un aspect avantageux, ledit procédé est une méthode par excès consistant :

1) à ajouter, audit milieu contenant l'analyte recherché, un premier réactif constitué par au moins un récepteur dudit analyte, couplé avec un composé fluorescent donneur constitué par un cryptate, un chélate ou un complexe macrocyclique de terre rare,
2) à ajouter un second réactif constitué par un ou plusieurs autres récepteurs dudit analyte, ledit second réactif étant couplé avec un composé fluorescent accepteur constitué par un complexe phycobiliprotéine-peptide de liaison,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

On pourra notamment utiliser dans lesdits procédés un seul récepteur de l'analyte, qui est couplé soit avec le composé fluorescent donneur, soit avec le composé fluorescent accepteur.

Dans un autre aspect de l'invention, ledit procédé est une méthode par compétition consistant :

1) à ajouter, audit milieu contenant l'analyte recherché, un premier réactif constitué par un récepteur dudit analyte, couplé avec un composé fluorescent donneur constitué par un cryptate, un chélate ou un complexe macrocyclique de terre rare,
2) à ajouter un second réactif constitué de l'analyte couplé avec un composé fluorescent accepteur constitué par un complexe phycobiliprotéine-peptide de liaison,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

Avantageusement, ledit procédé homogène de détection et/ou de détermination par fluorescence d'un analyte dans un milieu susceptible de le contenir, par mise en évidence du produit de la réaction entre l'analyte et au moins un récepteur correspondant, est une méthode par compétition consistant :

1) à ajouter, audit milieu contenant l'analyte recherché, un premier réactif constitué par un récepteur dudit analyte, ledit récepteur étant couplé avec un composé fluorescent accepteur constitué par un complexe phycobiliprotéine-peptide de liaison,
2) à ajouter un second réactif constitué de l'analyte couplé avec un composé fluorescent donneur constitué par un cryptate ou un chélate de terre rare,
3) à faire incuber ledit milieu soit après l'addition de chaque réactif, soit après l'addition des deux réactifs,
4) à exciter le milieu résultant à la longueur d'onde d'excitation du composé fluorescent donneur,
5) à mesurer le signal émis par le composé fluorescent accepteur.

Dans un aspect préféré des procédés mentionnés ci-dessus, le premier réactif et le second réactif sont ajoutés simultanément au milieu contenant l'analyte recherché.

Dans un aspect particulièrement avantageux selon l'invention, le composé fluorescent donneur utilisé dans les procédés mentionnés ci-dessus est un chélate, un cryptate ou un complexe macrocyclique de $Eu^{3+}$ et le composé fluorescent accepteur est le complexe $(\alpha^{AP}, \beta^{AP})_3$ LC.

Dans un aspect ultérieur, l'invention concerne également l'utilisation d'un complexe phycobiliprotéine-peptide de liaison tel que défini plus haut dans un procédé d'amplification du signal d'émission d'un cryptate ou d'un chélate de terre rare utilisé comme composé fluorescent donneur dans un dosage par fluorescence, dans lequel on met en oeuvre également un composé fluorescent accepteur, caractérisée en ce que le cryptate ou le chélate de terre rare possède un rendement quantique global faible et en ce que le rendement quantique de désactivation radiative du niveau émissif de la terre rare est inférieur au rendement quantique de l'accepteur, ledit accepteur étant constitué par ledit complexe phycobiliprotéine-peptide de liaison.

Dans un autre aspect, l'invention concerne également un conjugué fluorescent constitué d'un complexe phycobiliprotéine-peptide de liaison lié de manière covalente à l'un des éléments d'un couple de liaison spécifique ligand/récepteur.

Lesdits conjugués peuvent être en particulier utilisés pour trier les cellules et/ou analyser la surface cellulaire dans un procédé de cytométrie de flux, tel que décrit notament dans Mel. N.Kronic, J. Immunological Methods, 1986, 92, 1-13.

A titre d'exemples de couples de liaison spécifique ligand/récepteur, on peut notamment citer des couples protéine/protéine, protéine/ADN ou encore ADN/ADN.

De préférence, la phycobiliprotéine utilisée selon l'invention est choisie parmi la phycoerythrine, la phycoerythrocyanine, la phycocyanine, l'allophycocyanine et l'allophycocyanine B et le peptide de liaison est choisi parmi les peptides $L_R$, $L_C$, $L_{RC}$ et $L_{CM}$, tels que définis plus haut.

Avantageusement, le complexe phycobiliprotéine-peptide de liaison est choisi parmi les complexes

$$(\alpha^{PEC}, \beta^{PEC})_6 L_R, (\alpha^{PEC}, \beta^{PEC})_3 L_R,$$

$$(\alpha^{PC}, \beta^{PC})_6 L_R, (\alpha^{PC}, \beta^{PC})_6 L_{RC}, (\alpha^{PC}, \beta^{PC})_3 L_R,$$

$$(\alpha^{AP}, \beta^{AP})_3 L_C, (\alpha^{APB}, \alpha2^{AP}, \beta_3^{AP}) L_C \text{ et } (\alpha^{AP}, \beta^{AP})_2 L_{CM},$$

le complexe $(\alpha^{AP}, \beta^{AP})_3 L_C$ étant préféré.

De préférence, le complexe phycobiliprotéine-peptide de liaison est extrait d'une cyanobactérie choisie parmi *Mastigoclodus Laminosus, Synechocystis 6701, Synechococcus 6301, Anabaena variabilis* et *Nostoc spec.*.

Avantageusement, l'élément du couple de liaison spécifique ligand/récepteur est un récepteur, en particulier un récepteur cellulaire ou un anticorps. Ledit récepteur peut, par exemple, être l'avidine ou la streptavidine.

Dans un autre aspect avantageux, ledit élément est un ligand, notamment un analyte.Ledit ligand peut, par exemple, être un polypeptide, une lectine ou la biotine.

### <u>EXEMPLE</u> : Dosage de l'antigène carcinoembryonnaire (ACE)

Un immunoessai homogène a été réalisé en utilisant comme composé donneur le cryptate d'europium Eu trisbipyridine diamine préparé comme décrit dans la demande EP 321 353 (exemples 3 et 4) couplé à l'anticorps monoclonal G12 (CIS bio international, France) et comme composé accepteur soit l'allophycocyanine (Cyanotech, USA), soit le complexe $(\alpha^{AP}, \beta^{AP})_3 L_C$, couplées respectivement à l'anticorps monoclonal G15 (CIS bio international, France).

Les abréviations utilisées ci-après sont les suivantes :

AP = allophycocyanine
DTT = dithiotreitrol
EuTBP = cryptate d'europium Eu trisbipyridine diamine
BSA = sérum albumine bovine
HSA = serum albumine humaine
IgG = immunoglobuline G
SPDP = N-succinimidyl 3(2-pyridyldithio)propionate
Sulfo-SMCC = sulfosuccinimidyl 4-n-maléimidométhyl)cyclohexane

### 1) PREPARATION DES CONJUGUES IgG G15-AP

#### a) Activation de l'AP par le sulfo-SMCC

L'AP (3 mg) commercialement fournie sous forme précipitée dans une solution à 60 % de sulfate d'ammonium, est centrifugée. Après élimination du surnageant, le culot est repris par 250 µl de tampon phosphate 100 mM, pH 7,0, puis filtré à 0,8 µm afin d'éliminer les éventuelles particules en suspension.

Le filtrat est purifié par chromatographie d'exclusion sur colonne G25 superfine (Pharmacia, Suède) dans le même tampon. La concentration d'AP éluée dans le volume d'exclusion est déterminée à 650 nm, en considérant un

$\varepsilon_{650nm}$ de 731000M$^{-1}$ CM$^{-1}$.

L'activation de l'AP est réalisée en ajoutant une solution de sulfo-SMCC préparée extemporanément à raison de 6,9 mM dans un tampon phosphate 100 mM pH 7,0 et en laissant la réaction se produire pendant une heure, à température ambiante, sous agitation douce (rapport molaire de 15 à 75 sulfo-SMCC par AP). L'AP-maléimide est alors purifiée sur colonne G25 superfine en tampon phosphate 100 mM, EDTA 5mM, pH 6,5 et conservée à 4°C avant couplage sur IgG 3D3.

**b) Activation des IgG G15 par le SPDP**

Simultanément, 5mg d'IgG G15 à raison de 10 mg/ml dans un tampon phosphate 100 mM, pH 7,0 sont activés par l'ajout d'une solution de SPDP (Pierce, USA) à raison de 6,4 mM dans du dioxane dans un rapport molaire de 7,5 SPDP par IgG G15.

Après 35 min d'activation à température ambiante, l'IgG pyridine-2 thione est purifiée sur colonne G25 superfine dans un tampon phosphate 100 mM, EDTA 5mM, pH 6,5.

Les protéines sont concentrées et les groupes 2-pyridyl disulfides sont réduits par une solution de DTT (Sigma, USA) ayant une concentration finale de 19 mM pendant 15 min à température ambiante. Le DTT et la pyridine-2-thione sont éliminés par purification sur colonne G25 superfine en tampon phosphate 100 mM, EDTA 5mM, pH 6,5. La concentration en IgG-SH est déterminée à 280 nm avec un $\varepsilon_{280nm}$ de 210000 M$^{-1}$ cm$^{-1}$.

**c) Conjugaison des IgG G15-SH avec AP-maléimide**

La fixation des groupements thiols sur les maléimides est réalisée en ajoutant 2,51 mg d'AP activées par mg d'IgG G15-SH. Après 18 heures d'incubation à 4°C et à l'obscurité sous agitation douce, les fonctions thiols restées libres sont bloquées par l'addition d'une solution à 100 mM de N-méthyl maléimide (Sigma, USA) ayant une concentration finale de 20 mM pendant une heure à température ambiante.

Le milieu réactionnel est purifié par gel filtration sur colonne TSK G3000SW semi-préparative (Beckmann, USA) en tampon phosphate 100 mM pH 7,0.

Les concentrations en AP et en IgG G 15 du conjugué purifié, élué dans le premier pic, sont déterminées par les absorptions à 280 nm et à 650 nm, selon le calcul suivant :

$$[AP]_{Mole/1} = A_{650nM}/10000$$

$$[IgG]_{Mole/1} = (A_{280nm} - A'_{280nm}) / 210000$$

avec A'$_{280nm}$ étant la contribution à cette longueur d'onde de l'AP-maléimide, déterminée plus haut (paragraphe 1 - a) ).

De l'HSA est rajoutée à concurrence de 1 g/l au conjugué qui est ensuite réparti en aliquotes puis congelé à -20°C.

**2) PRÉPARATION DES CONJUGUES IgG G15-($\alpha^{AP}$, $\beta^{AP}$)$_3$L$_C$**

**a) Activation du complexe ($\alpha^{AP}$, $\beta^{AP}$)$_3$L$_C$, par le sulfo-SMCC.**

Le complexe ($\alpha^{AP}$, $\beta^{AP}$)$_3$L$_C$, est obtenu à partir de l'algue *Mastigocladus Laminosus* . Après extraction de phycobilisomes, les complexes protéines-peptides de liaison sont purifiés selon P. Fuglistaller et coll. Biol. Chem. Hoppe Seyler 1986, 367, 601-614.

Les complexes présentent les caractéristiques suivantes :

- $\varepsilon$ 650 = 1076000 M$^{-1}$ cm$^{-1}$.
- DO$_{650}$/DO$_{620}$ = 2,2.

A 1 ml de complexe ($\alpha^{AP}$, $\beta^{AP}$)$_3$L$_C$ à 3 mg/ml en tampon phosphate 100 mM pH 7,0, on ajoute 19,5 $\mu$l d'une solution de sulfo SMCC (Pierce) à 30 mmole/l dans le même tampon. On incube 30 mn à 30°C. Le produit de la réaction est purifié sur colonne G25 HR 10 x 10 débit 2ml/mn. La fraction éluée dans le volume mort est récupérée (V = 1,7 ml). La concentration en complexe ($\alpha^{AP}$, $\beta^{AP}$)L$_C$ maléimide est 1,4 mg/ml.

**b) Activation des IgG G15 par le SPDP**

Elle est réalisée comme indiquée au point 1, b) ci-dessus.

**c) Conjugaison des IgG G15-SH avec le complexe ($\alpha^{AP}$, $\beta^{AP}$)$_3$L$_C$-maléimide.**

1,7 ml de la solution obtenue au point 2, a est mise en contact avec 1 ml de la solution de IgG G15-SH à 0,9 mg/ml obtenue ci-dessus.

Après incubation une nuit à 4°C sous agitation par agitateur à rouleaux, le conjugué est purifié sur colonne TSK 4000 ½ préparative (Beckmann, USA) à débit de 4 ml/mn dans un tampon phosphate 100 mM pH7. Les fractions de 48 à 64 ml sont rassemblées et concentrées sur cône AMICON. La concentration en complexe ($\alpha^{AP}$, $\beta^{AP}$)$_3$L$_C$ en mg/ml est donnée par la formule DO650 / 10,76.

La concentration en IgG en mg/ml est donnée par la formule

$$DO_{280} - \frac{DO_{650}}{5,25} / 1,4.$$

Le conjugué est à 120 µl/ml en IgG avec un rapport molaire de 1,6 complexe ($\alpha^{AP}$, $\beta^{AP}$)$_3$L$_C$ / IgG DO$_{650}$ / 620 = 2,2.

**3) PRÉPARATION DES CONJUGUES IgG G12-Eu TBP**

La préparation de IgG G12-SH est réalisée selon le protocole décrit plus haut pour les G15 3D3 mais en faisant varier le rapport molaire de 4 à 16 SPDP par IgG G12.

A 5 mg (5 10$^{-6}$ moles) de Eu TBP est ajoutée une solution à 25 mM de sulfo-SMCC, en tampon phosphate 20 mM, diméthylformamide 10 % (v/v pH 7,0 dans une proportion de 2,5 moles d'activateur par mole de Eu TBP.

Après 45 min d'activation à température ambiante, le milieu réactionnel est filtré à 0,8 µm afin d'éliminer le précipité éventuellement formé. Les produits réactionnels indésirables (sulfo-SMCC, N-hydroxysuccinimide, acide (N-maléimidométhyl) carboxylique) sont éliminés par chromatographie échangeuse d'ions sur colonne Mono Q (Pharmacia, Suède) en tampon phosphate 20 mM diméthylformamide 10 % (v/v), pH 7,0 sous choc de NaCl. La concentration en Eu TBP maléimide est déterminée à 307 nm avec un $\varepsilon_{307nm}$ de 25000 M$^{-1}$ cm$^{-1}$ ainsi que le rapport A$_{307nm}$/A$_{280nm}$.

De façon similaire à celle décrite plus haut on fait réagir les fonctions maléimides avec les fonctions thiols fixés sur l'anticorps, dans des proportions molaires variant de 10 à 30 Eu TBP maléimide par IgG G12-SH.

Après 18 heures d'incubation à 4°C et blocage des groupements thiols (éventuellement restés libres) par N-méthylmaléimide, le Eu TBP non couplé est éliminé par dialyse en tampon phosphate 100 mM pH 7,0 à 4°C jusqu'à épuisement (plus de fluorescence dans les bains de dialyse).

Les caractéristiques du conjugué sont déterminées par ses absorptions à 307 nm et à 280 nm en utilisant les valeurs suivantes en tenant compte de l'absorption propre du cryptate déterminée par le rapport A$_{307nm}$/A$_{280nm}$.

**Eu TBP-maléimide :**

$\varepsilon_{307nm}$ = 25000 M$^{-1}$ cm$^{-1}$
A$_{307nm}$ /A$_{280nm}$ : déterminée expérimentalement.

**IgG G12-SH :**

$\varepsilon_{280nm}$ = 210000 M$^{-1}$ cm$^{-1}$
A$_{307nm}$ = 0 M$^{-1}$ cm$^{-1}$.

**4) APPLICATION AU DOSAGE DE L'ACE**

Les conjugués G12-Eu TBP, G15-AP et G15-complexe ($\alpha^{AP}$, $\beta^{AP}$)$_3$L$_C$ sont dilués dans un tampon phosphate 100 mM, pH 6, BSA 1 g/l, KF 400 mM.

On ajoute successivement dans des microplaques en polystyrène (Dynatech, USA):

- 100 µl de solution standard (sérum sans ACE) ou 100 µl d'échantillon à tester
- 100 µl de conjugué G12-Eu TBP à 0,5 µg/ml
- 100 µl de conjugué G15-AP à 5µg/ml ou 100 µl de conjugué G15-complexe ($\alpha^{AP}$, $\beta^{AP}$)$_3$L$_C$.

Après incubation pendant 3 h à 37°C on effectue la lecture à l'aide d'un fluorimètre à laser prototype, qui est décrit ci-après :

Un laser pulsé à azote (LASER SCIENCE INC., modèle LS1-337ND) est utilisé comme source d'excitation (longueur d'onde à 337,1 nm). La durée des pulsations est spécifiée à 3 nanosecondes et est répétée sous une fréquence de 10 Hertz. Le faisceau passe à travers un filtre (CORNING) afin d'éliminer toute lumière parasite à l'excitation autre que 337 nm.

Après être rentré dans la chambre de mesure, le faisceau est réfléchi par un filtre dichroïque, placé à 45 degrés, qui a la propriété de réfléchir les ultraviolets et de pouvoir transmettre la lumière visible.

Le faisceau réfléchi par le filtre dichroïque est focalisé sur le puits à mesurer d'une microplaque par une lentille en silice fondue. L'émission de fluorescence est collectée selon un angle solide de 20 degrés, collimatée par la même lentille, et passe directement à travers le filtre dichroïque (fluorescence en lumière visible).

Un filtre interférentiel, de caractéristiques définies selon la longueur d'onde de fluorescence à détecter, permet de se débarrasser des lumières pouvant parasiter le signal, dont l'intensité est ensuite mesurée par un photomultiplicateur (HAMAMATSU R2949).

Le compteur de photons utilisé est un SR-400 (STANFORD RESEARCH SYSTEMS), dont les opérations et la synchronisation avec le laser sont contrôlées par un ordinateur de type IBM PC-AT via une sortie RS 232. Les pulsations provenant du photomultiplicateur sont enregistrées pendant une fenêtre de temps ($t_g$) et après un délai ($t_d$) déterminés à condition qu'elles soient supérieures à un niveau discriminant sélectionné par le compteur de photons afin d'optimiser le rapport signal/bruit du photomultiplicateur.

Une table X-Y, pilotée par l'IBM PC-AT, permet les différents positionnements de la microplaque de mesure par des moteurs pas à pas, incluant les manoeuvres de chargement, de positionnement sous le faisceau excitant, de lecture automatique en séquentiel des 96 puits, et de sortie.

La fluorescence émise par le conjugué G15-AP ou G15-complexe ($\alpha^{AP}$, $\beta^{AP}$)$_3$L$_C$ est mesurée à l'aide du fluorimètre prototype équipé d'un filtre à 665 nm de 10 nm de largeur à mi-hauteur, pendant 400 µs et avec un délai de 50 µs.

Les résultats sont rapportés dans le tableau ci-dessous, exprimés en Δ cps correspondant à la différence entre le signal émis par l'échantillon à 665 nm par rapport à la solution standard (sérum sans ACE).

| ACE ng/ml | AP Δcps | Complexe ($\alpha^{AP}$, $\beta^{AP}$)$_3$L$_C$. Δcps |
|---|---|---|
| 4,4 | 251 | 388 |
| 15,9 | 966 | 1670 |
| 118 | 6427 | 9804 |
| 236 | 9436 | 16232 |

Les résultats montrent que le signal mesuré est supérieur pour le complexe phycobiliprotéine-protéine de liaison par rapport à la phycobiliprotéine seule.

De plus, on notera que le rapport $DO_{650}$/ $DO_{620}$ = 2,2 qui caractérise ledit complexe est constant dans la limite des erreurs de mesure pour toutes les étapes de la fabrication du conjugué et très différent de celui de l'AP ($\approx$ 1,45).

Ceci démontre la stabilité du complexe phycobiliprotéine-peptide de liaison tout au long des étapes de purification et de couplage effectuées sans précautions particulières par rapport à celle des conjugués G15 AP.

**Revendications**

1. Utilisation d'un complexe phycobiliprotéine-peptide de liaison de phycobilisome en tant que traceur fluorescent.

2. Utilisation d'un complexe phycobiliprotéine-peptide de liaison de phycobilisome en tant que traceur fluorescent dans un procédé de détection et/ou de détermination par fluorescence d'un analyte dans un milieu susceptible de le contenir.

**3.** Utilisation selon la revendication 1 ou 2, caractérisée en ce que la phycobiliprotéine est choisie parmi la phycoerythrine, la phycoerythrocyanine, la phycocyanine, l'allophycocyanine et l'allophycocyanine B.

**4.** Utilisation selon l'une des revendications 1,2 ou 3 caractérisée en ce que le complexe phycobiliprotéine-peptide de liaison est extrait d'une cyanobactérie choisie parmi *Mastigoclodus Laminosus, Synechocystis 6701, Synechococcus 6301, Anabaena variabilis* et *Nostoc spec.*

**5.** Utilisation selon l'une quelconque des revendications 1 à 4, caractérisée en ce que le peptide de liaison est choisi parmi les peptides $L_R$, $L_C$, $L_{RC}$ et $L_{CM}$.

**6.** Utilisation selon l'une quelconque des revendications 1 à 5, caractérisée en ce que le complexe phycobiliprotéine-peptide de liaison est choisi parmi les complexes

$(\alpha^{PEC}, \beta^{PEC})_6 L_R$, $(\alpha^{PEC}, \beta^{PEC})_3 L_R$,

$(\alpha^{PC}, \beta^{PC})_6 L_R$, $(\alpha^{PC}, \beta^{PC})_6 L_{RC}$, $(\alpha^{PC}, \beta^{PC})_3 L_R$,

$(\alpha^{AP}, \beta^{AP})_3 L_C$, $(\alpha^{APB}, \alpha 2^{AP}, \beta_3^{AP}) L_C$ et $(\alpha^{AP}, \beta^{AP})_2 L_{CM}$.

**7.** Procédé homogène de détection et/ou de détermination par fluorescence d'un analyte dans un milieu susceptible de le contenir, par mise en évidence du produit de la réaction entre l'analyte et au moins un récepteur correspondant, consistant :

1) à ajouter audit milieu un premier réactif constitué d'au moins un récepteur dudit analyte,
2) à ajouter un second réactif choisi parmi l'analyte ou au moins l'un de ses récepteurs, l'un des deux réactifs étant couplé avec un composé fluorescent donneur constitué par un cryptate, un chélate ou un complexe macrocyclique de terre rare et l'autre réactif étant couplé avec un composé fluorescent accepteur, l'ordre d'ajout des réactifs pouvant être inversé et, après excitation du mélange par une source de lumière à la longueur d'onde d'excitation du composé fluorescent donneur,
3) à mesurer le signal d'émission du composé fluorescent accepteur,

caractérisé en ce qu'on utilise comme composé fluorescent accepteur un complexe phycobiliprotéine-peptide de liaison tel que défini selon l'une quelconque des revendications 1 à 6.

**8.** Procédé selon la revendication 7 consistant en une méthode par excès, caractérisé en ce qu'il consiste :

1) à ajouter, audit milieu contenant l'analyte recherché, un premier réactif constitué par au moins un récepteur dudit analyte, couplé avec un composé fluorescent donneur constitué par un cryptate, un chélate ou un complexe macrocyclique de terre rare,
2) à ajouter un second réactif constitué par un ou plusieurs autres récepteurs dudit analyte, ledit second réactif étant couplé avec un composé fluorescent accepteur constitué par un complexe phycobiliprotéine-peptide de liaison,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

**9.** Procédé selon la revendication 7 consistant en une méthode par compétition, caractérisé en ce qu'il consiste :

1) à ajouter, audit milieu contenant l'analyte recherché, un premier réactif constitué par un récepteur dudit analyte, couplé avec un composé fluorescent donneur constitué par un cryptate, un chélate ou un complexe macrocyclique de terre rare,
2) à ajouter un second réactif constitué de l'analyte couplé avec un composé fluorescent accepteur constitué par un complexe phycobiliprotéine-peptide de liaison,
3) à faire incuber ledit milieu après chaque addition de réactifs ou après l'addition des deux réactifs,
4) à exciter le milieu résultant à la longueur d'onde d'excitation du composé fluorescent donneur,
5) à mesurer le signal émis par le composé fluorescent accepteur.

**10.** Procédé selon la revendication 7 consistant en une méthode par compétition, caractérisé en ce qu'il consiste :

1) à ajouter, audit milieu contenant l'analyte recherché, un premier réactif constitué par un récepteur dudit analyte, ledit récepteur étant couplé avec un composé fluorescent accepteur constitué par un complexe phycobiliprotéine-peptide de liaison,

2) à ajouter un second réactif constitué de l'analyte couplé avec un composé fluorescent donneur constitué par un cryptate ou un chélate de terre rare,

3) à faire incuber ledit milieu soit après l'addition de chaque réactif, soit après l'addition des deux réactifs,

4) à exciter le milieu résultant à la longueur d'onde d'excitation du composé fluorescent donneur,

5) à mesurer le signal émis par le composé fluorescent accepteur.

11. Procédé selon l'une quelconque des revendications 7 à 10, caractérisé en ce que le premier réactif et le second réactif sont ajoutés simultanément au milieu contenant l'analyte recherché.

12. Procédé selon l'une quelconque des revendications 7 ou 8, caractérisé en ce qu'on utilise un seul récepteur de l'analyte, qui est couplé soit avec le composé fluorescent donneur, soit avec le composé fluorescent accepteur.

13. Procédé selon l'une quelconque des revendications 7 à 12, caractérisé en ce que le composé fluorescent donneur est un chélate, un cryptate ou un complexe macrocyclique de terbium ou d'europium.

14. Procédé selon l'une quelconque des revendications 7 à 13, caractérisé en ce que le composé fluorescent donneur est un chélate, un cryptate ou un complexe macrocyclique de $Eu^{3+}$ et le composé fluorescent accepteur est le complexe $(\alpha^{AP}, \beta^{AP})_3\, L_C$.

15. Utilisation selon l'une quelconque des revendications 1 à 6 dans un procédé d'amplification du signal d'émission d'un cryptate ou d'un chélate de terre rare utilisé comme composé fluorescent donneur dans un dosage par fluorescence, dans lequel on met en oeuvre également un composé fluorescent accepteur, caractérisée en ce que le cryptate ou le chélate de terre rare possède un rendement quantique global faible et en ce que le rendement quantique de désactivation radiative du niveau émissif de la terre rare est inférieur au rendement quantique de l'accepteur, ledit accepteur étant constitué par un complexe phycobiliprotéine-peptide de liaison.

16. Utilisation selon l'une quelconque des revendications 1 à 6, caractérisée en ce que le complexe phycobiliprotéine-peptide de liaison est utilisé en combinaison avec un ou plusieurs autres traceurs fluorescents.

17. Conjugué fluorescent constitué d'un complexe phycobiliprotéine-peptide de liaison de phycobilisome lié de manière covalente à l'un des éléments d'un couple de liaison spécifique ligand/récepteur.

18. Conjugué selon la revendication 17, caractérisé en ce que la phycobiliprotéine est choisie parmi la phycoerythrine, la phycoerythrocyanine, la phycocyanine, l'allophycocyanine et l'allophycocyanine B.

19. Conjugué selon la revendication 17 ou 18, caractérisé en ce que le peptide de liaison est choisi parmi les peptides $L_R$, $L_C$, $L_{RC}$ et $L_{CM.}$.

20. Conjugué selon l'une des revendications 17 à 19, caractérisé en ce que le complexe phycobiliprotéine-peptide de liaison est choisi parmi les complexes

$(\alpha^{PEC}, \beta^{PEC})_6\, L_R$, $(\alpha^{PEC}, \beta^{PEC})_3\, L_R$,

$(\alpha^{PC}, \beta^{PC})_6\, L_R$, $(\alpha^{PC}, \beta^{PC})_6\, L_{RC}$, $(\alpha^{PC}, \beta^{PC})_3\, L_R$,

$(\alpha^{AP}, \beta^{AP})_3\, L_C$, $(\alpha^{APB}, \alpha_2^{AP}, \beta_3^{AP})\, L_C$ et $(\alpha^{AP}, \beta^{AP})_2\, L_{CM}$.

21. Conjugué selon l'une des revendications 17 à 20, caractérisé en ce que le complexe phycobiliprotéine-peptide de liaison est extrait d'une cyanobactérie choisie parmi *Mastigoclodus Laminosus, Synechocystis 6701, Synechococcus 6301, Anabaena variabilis* et *Nostoc spec.*.

22. Conjugué selon l'une des revendications 17 à 21, caractérisé en ce que l' élément du couple de liaison spécifique ligand/récepteur est un récepteur, en particulier un récepteur cellulaire ou un anticorps.

23. Conjugué selon l'une des revendications 17 à 21, caractérisé en ce que l' élément du couple de liaison spécifique ligand/récepteur est un ligand, notamment un analyte.

**24.** Utilisation d'un conjugué selon l'une quelconque des revendications 17 à 23 dans un procédé de cytométrie de flux.

**Patentansprüche**

**1.** Verwendung eines Phycobiliprotein-Phycobilisom-Bindungspeptid-Komplexes als fluoreszierender Tracer.

**2.** Verwendung eines Phycobiliprotein-Phycobilisom-Bindungspeptid-Komplexes als fluoreszierender Tracer in einem Verfahren zur Detektion und/oder zur Bestimmung eines Analyten in einem Medium, das diesen enthalten kann, durch Fluoreszenz.

**3.** Verwendung nach Anspruch 1 oder 2, dadurch gekennzeichnet, daß das Phycobiliprotein aus Phycoerythrin, Phycoerythrocyanin, Phycocyanin, Allophycocyanin und Allophycocyanin B ausgewählt wird.

**4.** Verwendung nach einem der Ansprüche 1, 2 oder 3, dadurch gekennzeichnet, daß der Phycobiliprotein-Bindungspeptid-Komplex aus einem Cyanobakterium extrahiert wird, das aus Mastigoclodus laminosus, Synechocystis 6701, Synechococcus 6301, Anabaena variabilis und Nostoc spec. ausgewählt wird.

**5.** Verwendung nach einem der Ansprüche 1 bis 4, dadurch gekennzeichnet, daß das Bindungspeptid aus den Peptiden $L_R$, $L_C$, $L_{RC}$ und $L_{CM}$ ausgewählt wird.

**6.** Verwendung nach einem der Ansprüche 1 bis 5, dadurch gekennzeichnet, daß der Phycobiliprotein-Bindungspeptid-Komplex aus den Komplexen

$(\alpha^{PEC}, \beta^{PEC})_6 L_R$, $(\alpha^{PEC}, \beta^{PEC})_3 L_R$

$(\alpha^{PC}, \beta^{PC})_6 L_R$, $(\alpha^{PC}, \beta^{PC})_6 L_{RC}$, $(\alpha^{PC}, \beta^{PC})_3 L_R$,

$(\alpha^{AP}, \beta^{AP})_3 L_C$, $(\alpha^{APB}, \alpha2^{AP}, \beta_3^{AP}) L_C$ et $(\alpha^{AP}, \beta^{AP})_2 L_{CM}$.

ausgewählt wird.

**7.** Homogenes Verfahren zur Detektion und/oder Bestimmung eines Analyten in einem Medium, das diesen enthalten kann, durch Fluoreszenz, indem das Produkt der Reaktion zwischen dem Analyten und zumindest einem entsprechenden Rezeptor nachgewiesen wird, welches Verfahren darin besteht, daß:

1) dem Medium ein erstes Reagenz zugesetzt wird, das aus zumindest einem Rezeptor des Analyten besteht,
2) ein zweites Reagenz, das aus dem Analyten oder zumindest einem seiner Rezeptoren ausgewählt wird, wobei eines der beiden Reagenzien mit einer fluoreszierenden Donor-Verbindung gekoppelt wird, die aus einem Cryptat, einem Chelat oder einem makrocyclischen Seltenerdkomplex besteht, und das andere Reagenz mit einer fluoreszierenden Akzeptor-Verbindung gekoppelt wird, und wobei die Reihenfolge der Reagenzien umgekehrt werden kann, nach Anregen der Mischung durch eine Lichtquelle mit einer Anregungswellenlänge der fluoreszierenden Donor-Verbindung zugesetzt wird,
3) das Emissionssignal der fluoreszierenden Akzeptor-Verbindung gemessen wird,

dadurch gekennzeichnet, daß als fluoreszierende Akzeptor-Verbindung ein Phycobiliprotein-Bindungspeptid-Komplex, wie in einem der Ansprüche 1 bis 6 definiert, verwendet wird.

**8.** Verfahren nach Anspruch 7, welches aus einer Überschußmethode besteht, dadurch gekennzeichnet, daß es darin besteht, daß:

1) dem den gesuchten Analyten enthaltenden Medium ein erstes Reagenz zugesetzt wird, bestehend aus zumindest einem Rezeptor des Analyten, gekoppelt mit einer fluoreszierenden Donor-Verbindung, die aus einem Cryptat, einem Chelat oder einem makrocyclischen Seltenerdkomplex besteht,
2) ein zweites Reagenz zugesetzt wird, bestehend aus einem oder mehreren anderen Rezeptoren des Analyten, welches zweite Reagenz mit einer fluoreszierenden Akzeptor-Verbindung gekoppelt wird, die aus einem Phycobiliprotein-Bindungspeptid-Komplex besteht,

3) das Medium nach jedem Reagenzien-Zusatz oder nach dem Zusatz der beiden Reagenzien inkubiert wird,

4) das erhaltene Medium bei der Anregungswellenlänge der fluoreszierenden Donor-Verbindung angeregt wird,

5) das von der fluoreszierenden Akzeptor-Verbindung emittierte Signal gemessen wird.

9. Verfahren nach Anspruch 7, welches aus einer Konkurrenzmethode besteht, dadurch gekennzeichnet, daß es darin besteht, daß:

1) dem den gesuchten Analyten enthaltenden Medium ein erstes Reagenz zugesetzt wird, bestehend aus einem Rezeptor des Analyten, gekoppelt mit einer fluoreszierenden Donor-Verbindung, die aus einem Cryptat, einem Chelat oder einem makrocyclischen Seltenerdkomplex besteht,

2) ein zweites Reagenz zugesetzt wird, bestehend aus dem Analyten, gekoppelt mit einer fluoreszierenden Akzeptor-Verbindung, die aus einem Phycobiliprotein-Bindungspeptid-Komplex besteht,

3) das Medium nach jedem Reagenzien-Zusatz oder nach dem Zusatz der beiden Reagenzien inkubiert wird,

4) das erhaltene Medium bei der Anregungswellenlänge der fluoreszierenden Donor-Verbindung angeregt wird,

5) das von der fluoreszierenden Akzeptor-Verbindung emittierte Signal gemessen wird.

10. Verfahren nach Anspruch 7, welches aus einer Konkurrenzmethode besteht, dadurch gekennzeichnet, daß es darin besteht, daß:

1) dem den gesuchten Analyten enthaltenden Medium ein erstes Reagenz zugesetzt wird, bestehend aus einem Rezeptor des Analyten, welcher Rezeptor mit einer fluoreszierenden Akzeptor-Verbindung gekoppelt wird, die aus einem Phycobiliprotein-Bindungspeptid-Komplex besteht,

2) ein zweites Reagenz zugesetzt wird, bestehend aus dem Analyten, gekoppelt mit einer fluoreszierenden Donor-Verbindung, die aus einem Cryptat oder einem Seltenerdchelat besteht,

3) das Medium nach dem Zusatz jedes Reagenz oder nach dem Zusatz der beiden Reagenzien inkubiert wird,

4) das erhaltene Medium bei der Anregungswellenlänge der fluoreszierenden Donor-Verbindung angeregt wird,

5) das von der fluoreszierenden Akzeptor-Verbindung emittierte Signal gemessen wird.

11. Verfahren nach einem der Ansprüche 7 bis 10, dadurch gekennzeichnet, daß das erste Reagenz und das zweite Reagenz gleichzeitig dem den gesuchten Analyten enthaltenden Medium zugesetzt werden.

12. Verfahren nach einem der Ansprüche 7 oder 8, dadurch gekennzeichnet, daß ein einzelner Rezeptor des Analyten verwendet wird, der entweder mit der fluoreszierenden Donor-Verbindung oder mit der fluoreszierenden Akzeptor-Verbindung gekoppelt wird.

13. Verfahren nach einem der Ansprüche 7 bis 12, dadurch gekennzeichnet, daß die fluoreszierende Donor-Verbindung ein Chelat, ein Cryptat oder ein makrocyclischer Terbium- oder Europium-Komplex ist.

14. Verfahren nach einem der Ansprüche 7 bis 13, dadurch gekennzeichnet, daß die fluoreszierende Donor-Verbindung ein Chelat, ein Cryptat oder ein $Eu^{3+}$-Komplex ist, und die fluoreszierende Akzeptor-Verbindung der Komplex $(\alpha^{AP}, \beta^{AP})_3 L_C$ ist.

15. Verwendung nach einem der Ansprüche 1 bis 6 in einem Verfahren zur Verstärkung des Emissionssignals eines Cryptats oder eines Seltenerdchelats, das als fluoreszierende Donor-Verbindung in einer Fluoreszenzmessung verwendet wird, wobei auch eine fluoreszierende Akzeptor-Verbindung verwendet wird, dadurch gekennzeichnet, daß das Cryptat oder Seltenerdchelat eine schwache quantitative Gesamtausbeute aufweist, und daß die quantitative Ausbeute der Strahlendeaktivierung des Emissionsniveaus der Seltenerde geringer ist als die quantitative Ausbeute des Akzeptors, welcher Akzeptor aus einem Phycobiliprotein-Bindungspeptid-Komplex besteht.

16. Verwendung nach einem der Ansprüche 1 bis 6, dadurch gekennzeichnet, daß der Phycobiliprotein-Bindungspeptid-Komplex in Kombination mit einem oder mehreren anderen fluoreszierenden Tracern verwendet wird.

17. Fluoreszierendes Konjugat, welches aus einem Phycobiliprotein-Phycobilisom-Bindungspeptid-Komplex besteht, der kovalent mit einem der Elemente eines spezifischen Bindungspaares Ligand/Rezeptor gebunden ist.

**18.** Konjugat nach Anspruch 17, dadurch gekennzeichnet, daß das Phycobiliprotein aus Phycoerythrin, Phycoerythrocyanin, Phycocyanin, Allophycocyanin und Allophycocyanin B ausgewählt wird.

**19.** Konjugat nach Anspruch 17 oder 18, dadurch gekennzeichnet, daß das Bindungspeptid aus den Peptiden $L_R$, $L_C$, $L_{RC}$ und $L_{CM}$ ausgewählt wird.

**20.** Konjugat nach einem der Ansprüche 17 bis 19, dadurch gekennzeichnet, daß der Phycobiliprotein-Bindungspeptid-Komplex aus den Komplexen

$(\alpha^{PEC}, \beta^{PEC})_6 L_R$, $(\alpha^{PEC}, \beta^{PEC})_3 L_R$,

$(\alpha^{PC}, \beta^{PC})_6 L_R$, $(\alpha^{PC}, \beta^{PC})_6 L_{RC}$, $(\alpha^{PC}, \beta^{PC})_3 L_R$,

$(\alpha^{AP}, \beta^{AP})_3 L_C$, $(\alpha^{APB}, \alpha_2^{AP}, \beta_3^{AP}) L_C$ et $(\alpha^{AP}, \beta^{AP})_2 L_{CM}$.

ausgewählt wird.

**21.** Konjugat nach einem der Ansprüche 17 bis 20, dadurch gekennzeichnet, daß der Phycobiliprotein-Bindungspeptid-Komplex aus einem Cyanobakterium extrahiert wird, das aus Mastigoclodus laminosus, Synechocystis 6701, Synechococcus 6301, Anabaena variabilis und Nostoc spec. ausgewählt wird.

**22.** Konjugat nach einem der Ansprüche 17 bis 21, dadurch gekennzeichnet, daß das Element des spezifischen Bindungspaares Ligand/Rezeptor ein Rezeptor, insbesondere ein Zellrezeptor, oder ein Antikörper ist.

**23.** Konjugat nach einem der Ansprüche 17 bis 21, dadurch gekennzeichnet, daß das Element des spezifischen Bindungspaares Ligand/Rezeptor ein Ligand, insbesondere ein Analyt, ist.

**24.** Verwendung eines Konjugats nach einem der Ansprüche 17 bis 23 in einem Flußcytometrie-Verfahren.

**Claims**

**1.** Use of a phycobiliprotein-phycobilisome linker peptide complex as a fluorescent tracer.

**2.** Use of a phycobiliprotein-phycobilisome linker peptide complex as a fluorescent tracer in a fluorescent method of detecting and/or determining an analyte in a medium in which it may be present.

**3.** Use according to claim 1 or 2, characterised in that the phycobiliprotein is selected from phycoerythrin, phycoerythrocyanine, phycocyanine, allophycocyanine and allophycocyanine B.

**4.** Use according to one of claims 1, 2 or 3 characterised in that the phycobiliprotein-linker peptide complex is extracted from a cyanobacterium selected from *Mastigoclodus Laminosus, Synechocystis 6701, Synechococcus 6301, Anabaena variabilis* and *Nostoc spec.*

**5.** Use according to any one of claims 1 to 4, characterised in that the linker peptide is selected from the peptides $L_R$, $L_C$, $L_{RC}$ and $L_{CM}$.

**6.** Use according to any one of claims 1 to 5, characterised in that the phycobiliprotein-linker peptide complex is selected from the complexes

$(\alpha^{PEC}, \beta^{PEC})_6 L_R$, $(\alpha^{PEC}, \beta^{PEC})_3 L_R$,

$(\alpha^{PC}, \beta^{PC})_6 L_R$, $(\alpha^{PC}, \beta^{PC})_6 L_{RC}$, $(\alpha^{PC}, \beta^{PC})_3 L_R$,

$(\alpha^{AP}, \beta^{AP})_3 L_C$, $(\alpha^{APB}, \alpha2^{AP}, \beta_3^{AP}) L_C$ and $(\alpha^{AP}, \beta^{AP})_2 L_{CM}$.

**7.** A homogeneous fluorescent method of detecting and/or determining an analyte in a medium in which it may be present, by displaying the product of the reaction between the analyte and at least one corresponding receptor, consisting :

1) in adding, to said medium, a first reagent constituted of at least one receptor of said analyte,

2) in adding a second reagent selected from the analyte or at least one of its receptors, one of the two reagents being coupled with a fluorescent donor compound constituted by a rare earth cryptate, chelate or macrocyclic complex and the other reagent being coupled with a fluorescent acceptor compound, it being possible for the order of addition of the reagents to be the reverse order and, after excitation of the mixture by a source of light at the excitation wavelength of the fluorescent donor compound,

3) in measuring the emission signal of the fluorescent acceptor compound, characterised in that a phycobiliprotein-linker peptide complex such as defined according to any one of claims 1 to 6 is used as fluorescent acceptor compound.

8. The method according to claim 7 which consists of an excess method, characterised in that it consists :

1) in adding, to said medium containing the analyte sought after, a first reagent constituted by at least one receptor of said analyte coupled with a fluorescent donor compound constituted by a rare earth cryptate, chelate or macrocyclic complex,

2) in adding a second reagent constituted by one or more other receptors of said analyte, said second reagent being coupled with a fluorescent acceptor compound constituted by a phycobiliprotein-linker peptide complex,

3) in incubating said medium after each addition of reagents or after the addition of the two reagents,

4) in exciting the resulting medium at the excitation wavelength of the fluorescent donor compound,

5) in measuring the signal emitted by the fluorescent acceptor compound.

9. The method according to claim 7 consisting of a competitive method, characterised in that it consists :

1) in adding, to said medium containing the analyte sought after, a first reagent constituted by a receptor of said analyte, coupled with a fluorescent donor compound constituted by a rare earth cryptate, chelate or macrocyclic complex,

2) in adding a second reagent constituted of the analyte coupled with a fluorescent acceptor compound constituted by a phycobiliprotein-linker peptide complex,

3) in incubating said medium after each addition of reagents or after the addition of the two reagents,

4) in exciting the resulting medium at the excitation wavelength of the fluorescent donor compound,

5) in measuring the signal emitted by the fluorescent acceptor compound.

10. The method according to claim 7 consisting of a competitive method, characterised in that it consists :

1) in adding, to said medium containing the analyte sought after, a first reagent constituted by a receptor of said analyte, said receptor being coupled with a fluorescent acceptor compound constituted by a phycobiliprotein-linker peptide complex,

2) in adding a second reagent constituted of the analyte coupled with a fluorescent donor compound constituted by a rare earth cryptate or chelate,

3) in incubating said medium either after the addition of each reagent, or after the addition of the two reagents,

4) in exciting the resulting medium at the excitation wavelength of the fluorescent donor compound,

5) in measuring the signal emitted by the fluorescent acceptor compound.

11. The method according to any one of claims 7 to 10, characterised in that the first reagent and the second reagent are added simultaneously to the medium containing the analyte sought after.

12. The method according to any one of claims 7 or 8, characterised in that one sole receptor of the analyte is used which is coupled either with the fluorescent donor compound, or with the fluorescent acceptor compound.

13. The method according to any one of claims 7 to 12, characterised in that the fluorescent donor compound is a terbium or europium chelate, cryptate or macrocyclic complex.

14. The method according to any one of claims 7 to 13, characterised in that the fluorescent donor compound is an $Eu^{3+}$ chelate, cryptate or macrocyclic complex and the fluorescent acceptor compound is the $(\alpha^{AP},\beta^{AP})_3 L_C$ com-plex.

**15.** Use according to any one of claims 1 to 6 in a method of amplification of the emission signal of a rare earth cryptate or chelate used as fluorescent donor compound in a determination by fluorescence, in which a fluorescent acceptor compound is also used, characterised in that the rare earth cryptate or chelate possesses a low overall quantum yield, and in that the quantum yield of radiative deactivation from the emission level of the rare earth is lower than the quantum yield of the acceptor, said acceptor being constituted by a phycobiliprotein-linker peptide complex.

**16.** Use according to any one of claims 1 to 6, characterised in that the phycobiliprotein-linker peptide complex is used in combination with one or more different fluorescent tracers.

**17.** A fluorescent conjugate constituted of a phycobiliprotein-phycobilisome linker peptide complex covalently bound to one of the elements of a ligand/receptor specific binding pair.

**18.** The conjugate according to claim 17, characterised in that the phycobiliprotein is selected from phycoerythrin. phycoerythrocyanine, phycocyanine, allophycocyanine and allophycocyanine B.

**19.** The conjugate according to claim 17 or 18, characterised in that the linker peptide is selected from the peptides $L_R$, $L_C$, $L_{RC}$ and $L_{CM}$.

**20.** The conjugate according to one of claims 17 to 19, characterised in that the phycobiliprotein-linker peptide complex is selected from the complexes

$$(\alpha^{PEC}, \beta^{PEC})_6 \, L_R, \; (\alpha^{PEC}, \beta^{PEC})_3 \, L_R,$$

$$(\alpha^{PC}, \beta^{PC})_6 \, L_R, \; (\alpha^{PC}, \beta^{PC})_6 \, L_{RC}, \; (\alpha^{PC}, \beta^{PC})_3 \, L_R,$$

$$(\alpha^{AP}, \beta^{AP})_3 \, L_C, \; (\alpha^{APB}, \alpha_2^{AP}, \beta_3^{AP}) \, L_C \text{ and } (\alpha^{AP}, \beta^{AP})_2 \, L_{CM}.$$

**21.** The conjugate according to one of claims 17 to 20, characterised in that the phycobiliprotein-linker peptide complex is extracted from a cyanobacterium selected from *Mastigoclodus Laminosus, Synechocystis 6701, Synechococcus 6301, Anabaena variabilis* and *Nostoc spec.*.

**22.** The conjugate according to one of claims 17 to 21, characterised in that the element of the ligand/receptor specific binding pair is a receptor, in particular a cell receptor or an antibody.

**23.** The conjugate according to one of claims 17 to 21, characterised in that the element of the ligand/receptor specific binding pair is a ligand, notably an analyte.

**24.** Use of a conjugate according to any one of claims 17 to 23 in a flow cytometry method.